# FASCICULE DE BREVET EUROPEEN

(11) **EP 0 955 042 B1**
(45) Date de publication et mention de la délivrance du brevet: **25.06.2003**
(21) Numéro de dépôt: 99401011.4
(22) Date de dépôt: 26.04.1999
(51) Int. Cl.: A61K 9/48, A61K 9/10, A61K 31/57, A61K 31/565

(54) **Composition pharmaceutique à base d'estrogène et de progestérone**
Pharmazeutische Zusammensetzung von Estrogen und Progesteron
Pharmaceutical composition of estrogen and progesteron

(30) Priorité: 27.04.1998 FR 9805262
(43) Date de publication de la demande: 10.11.1999
(73) Titulaire: Laboratoires EFFIK, 91571 Bievres Cédex (FR)
(72) Inventeur: Galdona Gil, Javier, 28033 Madrid (ES); Grasset, Etienne, 92100 Boulogne (FR); Terracol, Didier, 91370 Verrieres Le Buisson (FR)
(74) Mandataire: Casalonga, Axel

(56) Documents cités:
- EP-A- 0 393 539
- EP-A- 0 461 290
- WO-A-94/22426
- WO-A-97/40823
- FR-A- 2 747 042
- US-A- 4 383 993
- US-A- 4 900 734

## Description

La présente invention est relative à une composition à base de progestérone et d'estrogène, destinée au traitement hormonal substitutif de la ménopause.

L'utilisation du traitement hormonal substitutif (THS) estroprogestatif de la carence hormonale postménopausique est très développée en Europe en particulier et d'ampleur sans cesse croissante.

Ce développement est dû à plusieurs facteurs :
- l'évolution démographique,
- l'augmentation de la durée de vie qui le rend de plus en plus nécessaire,
- les démonstrations de plus en plus nombreuses de ses bienfaits à des niveaux très variés,
- la mise au point de schémas thérapeutiques en termes de doses et de rythmes d'administration, permettant d'administrer la dose minimum efficace en évitant ou en minimisant les hémorragies de privation et le risque endométrial.

Le cycle hormonal de la femme est dû essentiellement à la production de deux hormones, le 17β-estradiol [(17β)-estra-1,3,5(10)-triéno-3,17-diol] et la progestérone [α4-pregneno-3,20-dione]. En cas de cycle déficient, en particulier pendant la ménopause où la production endogène des deux hormones diminue, il est habituel de compenser cette déficience par une supplémentation exogène de ces deux hormones définie comme traitement hormonal substitutif.

Le traitement hormonal substitutif de la ménopause implique l'administration d'un estrogène. Cet estrogène peut être administré par différentes voies, notamment orales ou transdermiques. Les substances utilisées dans le cas de l'administration orale sont, soit l'hormone naturelle (17β-estradiol) ou le valérate de 17β-estradiol, généralement micronisés, soit les dérivés conjugués équins, soit différents dérivés (estriol, oestrone). Les posologies quotidiennes d'estrogènes les plus fréquentes varient de 1 à 2 mg de 17β-estradiol ou de 1 à 2 mg de valérate de 17β-estradiol (ce qui correspond à 0,76 et 1,53 mg d'estradiol base respectivement), alors que pour les dérivés conjugués équins, les posologies quotidiennes varient de 0,625 mg à 1,250 mg. L'efficacité à long terme de doses inférieures d'estrogène par voie orale n'a pas été démontrée avec certitude. (Rozenbaum : "Oestrogènes in : Pharmacologie Clinique" : Giroud, Mahé, Meyniel. Eds. Expansion Scientifique Française, Paris, 1988, 2075-2092).

L'administration systémique prolongée et isolée d'estrogènes augmente le risque d'une hyperplasie de l'endomètre. La survenue d'une hyperplasie atypique expose la femme à un risque accrû de cancer de l'endomètre. Pour diminuer ce risque, il est habituel d'accompagner le traitement estrogénique d'un traitement progestatif. Pour ce traitement complémentaire, il peut être utilisé des progestatifs de synthèse ou l'hormone naturelle (progestérone), et de préférence cette dernière, car son métabolisme apporte des avantages supplémentaires : la progestérone n'ayant pas d'effet androgénique, elle n'induit pas la baisse de taux de certaines lipoprotéines, comme par exemple le HDLC cholestérol (Bolaji, Grimes, Mortimer, Tallon, Fottrell, O'dwyer : "Low dose progestérone therapy in oestrogenised postmenopausal women : effects on plasma lipids, lipoproteins and liver function parameters", Eur. J. Obstet. Gynecol. Reprod. Biol. 1993,48:61-8; et The Writing Group For The Pepi Trial : "Effects of estrogen/progestin regimens on heart disease risk factors in postmenopausal women", Jama, 1995, 273 : 195-208).

Pour l'administration de progestérone par voie orale, les posologies les plus souvent utilisées dans le traitement hormonal substitutif de la ménopause varient de 100 à 300 mg par jour. Ces posologies permettent de protéger efficacement l'endomètre (Darj, Nilsson, Axelsson, Hellberg : "Clinical and endometrial effects of oestradiol and progesterone in postmenopausal women", Maturitas, 1991, 13 : 109-15; et Faguer, Gillet, André, Philippe : "Aménorrhée lors du traitement hormonal substitutif de la ménopause par l'association d'estradiol percutané et de progestérone orale micronisée", Contracep. Fertil. Sex., 1993, 231 : 849-52; et Moyer, De Lignières, Driguez, Pez : Prevention of endometrial hyperplasia by progesterone during long term estradiol replacement : influence of bleeding pattern and secretory changes", Fertil. Steril., 1993, 59 : 992-7).

D'autre part, il est connu que l'utilisation de la progestérone micronisée en suspension dans un excipient huileux améliore son absorption per os (Hargrove, Maxson, Colston, Wentz : "Absorption of oral progesterone is influenced by vehicle and particule size", Am. J. Obstet. Gynecol., 1989, 161 : 948-51). Dans ces conditions, il a été possible d'obtenir des concentrations efficaces de progestérone dans les organes cibles (Morville, Dray, Reynier, Barrat : "Biodisponibilité de la progestérone naturelle administrée par voie orale. Mesure des concentrations du stéroïde dans le plasma, l'endomètre et le tissu mammaire", J. Gyn. Obstet. Biol. Reprod., 1992, 11 : 355-63).

Il existe sur le marché des capsules molles pour l'administration par voie orale ou vaginale de progestérone naturelle micronisée en suspension dans de l'huile d'arachide, comme par exemple les capsules commercialisées par les Laboratoires Besins-Iscovesco en France, sous la marque Utrogestan®.

La difficulté de fabriquer un médicament associant les deux hormones aux doses souhaitées (progestérone et 17β-estradiol ou un sel ou un dérivé de ce sel) dans une seule forme galénique, oblige à ce que le traitement hormonal substitutif de la ménopause habituel comprenne l'administration de deux médicaments distincts, l'un apportant l'estrogène et l'autre apportant la progestérone ou un autre progestatif.

L'utilisation de deux médicaments séparés pour traiter la ménopause renchérit le coût du traitement, affecte la bonne utilisation, et porte en soi un risque certain de non suivi thérapeutique qui peut provoquer une diminution de l'efficacité thérapeutique attendue du traitement.

Par conséquent, un médicament qui contient les deux hormones dans une seule forme galénique est souhaitable.

L'association des deux hormones dans une même forme pharmaceutique est un facteur de sécurité et garantit le suivi du traitement, puisqu'il devient matériellement impossible que la patiente ne prenne que l'estrogène seul sans son complément correspondant en progestatif.

Les inventeurs ont découvert, ce qui fait l'objet de l'invention, qu'il est possible de préparer un médicament contenant les deux hormones sous une seule forme galénique et libérant dans le corps ces hormones de façon équilibrée..

On a déjà proposé, voir FR-A-2 747 042, des médicaments à base de progestérone et d'estradiol, consistant à introduire dans une suspension de progestérone à l'état micronisé dans un milieu à base d'huile et l'estradiol, en évitant cependant que l'estradiol ne se dissolve, même partiellement dans l'huile. Il prévoit de renfermer l'estradiol dans une microsphère constituée d'un polymère insoluble dans l'huile et soluble dans le milieu biologique. Ce type de composition n'est pas totalement satisfaisant et pose notamment des problèmes d'hétérogénéité du fait des granulométries et des densités différentes des substances actives présentes sous forme particulaire.

On connaît également par EP-A-0393539, des préparations orales conditionnées dans des capsules de gélatine molle contenant du valérate d'estradiol dissous dans un milieu lipophile physiologiquement acceptable tel qu'une huile végétale.

Ce brevet constate que cette forme de préparation conduit à une biodisponibilité plus élevée et à une accélération de la libération de la substance active.Il est envisagé la mise en oeuvre d'une composition contenant sous forme dissoute le valérate d'estradiol et un progestatif dans une proportion de 5 mg/1 mg de l'estrogène également dissous dans le milieu lipophile.

La demande WO 97/40823 a pour objet des compositions pouvant contenir la progestérone, de l'estradiol et de la testostérone. Ce document vise également à utiliser les substances actives sous forme dissoute dans le milieu et, dans ce but met en oeuvre une combinaison d'un agent de surface lipophile et d'un agent de surface hydrophile susceptibles d'améliorer la solubilisation de l'estrogène et de la progestérone dans un milieu lipophile. La concentration maximale en progestérone dans un milieu ne contenant pas d'alcool, est de 4,5%.

La demande WO 94/22426 décrit des compositions contenant le 17β-estradiol et de la progestérone destinées à être administrées par les muqueuses, notamment par voie nasale. Ces 2 composés sont dissous dans une huile, mélangée à une phase aqueuse afin de préparer l'émulsion. Celle-ci assure une libération rapide des hormones présentes dans les goutellettes lipophiliques.

Le brevet US 4383993 a pour objet des compositions nasales contenant de la progestérone et du 17β-estradiol, éventuellement sous forme de valérate, dans une composition différente de celle revendiquée, destinées à la contraception de mammifères.

Le brevet US 4900734 a pour objet une composition pharmaceutique contenant du 17β-estradiol dans un véhicule huileux comprenant une suspension de particules de progestérone micronisée.

Les inventeurs ont découvert qu'il était possible de mettre à la disposition des patientes, nécessitant un traitement hormonal substitutif de la ménopause, une composition comprenant un estrogène en solution dans le milieu lipophile d'une suspension de progestérone.

Ils ont pu constater en particulier que cette combinaison permettait d'administrer la progestérone et l'estrogène simultanément tout en maintenant l'équilibre entre l'estrogène et la progestérone nécessaire à un traitement hormonal substitutif de la ménopause efficace, c'est-à-dire libérant l'estrogène et la progestérone dans les proportions relatives souhaitées Le traitement avec la combinaison selon l'invention permet de substituer les hormones naturelles et réduire les bouffées de chaleur, les troublés génito-urinaires, les risques d'ostéoporose tout en protégeant efficacement l'endomètre.

Les inventeurs ont également découvert à leur grande surprise que la solubilisation totale du valérate d'estradiol dans le milieu de la suspension de progestérone permettait d'obtenir une libération d'estradiol plus lente, du fait de la proportion relativement élevée de progéstérone sous forme particulaire, et donc prolongée dans le temps contrairement à ce que l'on pouvait déduire de l'état de la technique antérieure.

Un objet de l'invention est donc une composition pharmaceutique comprenant du valérate d'estradiol en solution dans le milieu lipophile d'une suspension de progestérone.

Un autre objet de l'invention est constitué par le procédé de préparation de cette composition.

L'invention a également pour objet l'utilisation de cette composition pour le traitement substitutif hormonal de la ménopause.

D'autres objets de l'invention apparaîtront à la lecture de la description des exemples qui suivent.

La composition conforme à l'invention destinée à être utilisée dans le traitement hormonal substitutif de la ménopause comprend une suspension de progestérone dans un milieu lipophile et du valérate de 17β-estradiol solubilisé dans le milieu lipophile, le rapport en poids de progestérone sur le poids en 17β-estradiol base correspondant, étant compris entre 25 et 600.

La progestérone est utilisée de préférence sous forme micronisée, la granulométrie des particules étant généralement comprise entre 1 et 100 microns et de préférence entre 1 et 50 microns.

La progestérone est essentiellement en suspension, bien qu'il puisse arriver qu'une partie relativement faible ne dépassant généralement pas 5% puisse éventuellement être dissoute dans le milieu lipophile.

L'estrogène doit être totalement dissout dans le milieu lipophile.

Le milieu lipophile est constitué par des huiles, de préférence d'origine végétale, habituellement utilisées en galénique telles que l'huile d'arachide, l'huile de sésame, l'huile de soja, l'huile de coprah fractionnée.

On peut ajouter à ce milieu un agent tensio-actif, notamment les agents tensio-actifs amphotères tels-que de la lécithine de soja. Ces agents tensio-actifs peuvent être utilisés dans des proportions de 0,05 à 5% en poids par rapport au poids d'huile.

Les compositions conformes à l'invention contiennent de préférence entre 50 et 300 mg de progestérone en suspension et de 0,5 à 2 mg de 17β-estradiol base (sous la forme de valérate de 17β-estradiol).

Les compositions conformes à l'invention sont préparées en procédant dans une première étape à la dissolution de l'estrogène dans le milieu lipophile. On ajoute ensuite la progestérone sous forme de particules micronisées dans la solution lipophile d'estrogène et l'on procède au mélange de la progestérone sous forme de particules dans ladite solution lipophile d'estrogène.

Conformément à l'invention, l'estrogène est totalement dissous dans le milieu lipophile, la progestérone, quant à elle, se retrouvant essentiellement en suspension.

Les inventeurs ont découvert qu'il était possible de dissoudre totalement sans risque de recristallisation, l'estrogène et plus particulièrement le valérate de 17β-estradiol dans le milieu lipophile défini ci-dessus, en chauffant le mélange à une température susceptible de provoquer la dissolution totale du valérate d'estradiol, en particulier comprise entre 30 et 50°, et de préférence aux environs de 40°C.

La dissolution totale de l'estrogène constitue un élément important de l'invention, dans la mesure où la subsistance de particules d'estrogène en présence des particules de progestérone pouvait entraîner des problèmes d'hétérogénéité des capsules en raison des dimensions et des densités différentes des particules et de la présence largement majoritaire des particules de progestérone dans la composition.

Les inventeurs ont également pu noter que la présence de progestérone sous forme de particules dans la composition à titre majoritaire contribuait à régulariser la cinétique de libération de l'estrogène, permettant de ce fait la mise à disposition d'un traitement équilibré.

Les compositions conformes à l'invention peuvent être formulées sous des formes galéniques appropriées, permettant de maintenir la suspension en milieu lipophile de l'invention.

Les compositions peuvent être formulées pour une administration par voie orale ou vaginale.

Une préparation particulière et préférentielle de l'invention est une formulation en capsules molles. Ces capsules sont obtenues par des techniques conventionnelles [Tratado de Farmacia Galénica, C. Fauli i Trillo, Luzan 5 S.A. de Ediciones, I^{a} Edicion, (1993), pages 497 et suivantes, spécialmente pages 588-607].

Ces capsules molles peuvent être réalisées en gélatine ou toute autre substance habituellement utilisée pour réaliser de telles capsules molles.

Les compositions conformes à l'invention sont utilisées pour le traitement hormonal substitutif de la ménopause, qui consiste à administrer à une patiente potentielle une quantité thérapeutiquement efficace d'une composition conforme à l'invention.

L'expression "quantité thérapeutiquement efficace" signifie la quantité de la composition pharmaceutique suffisante pour traiter la ménopause.

On utilise en général des doses actives de 0,5 à 2 mg/jour de 17β-estradiol base sous la forme, notamment le valérate de 17β-estradiol, et 50 à 300 mg/jour de progestérone.

Selon une forme de réalisation préférentielle de l'invention, on utilise des capsules molles contenant 100 mg de progestérone micronisée et 1 mg de valérate d'estradiol, administrées par voie orale ou vaginale une fois par jour.

Cette posologie peut cependant varier en fonction de la pathologie concernée, de la sévérité, de la durée du traitement, du poids et de l'âge de la patiente et de l'efficacité relative de la composition.

Grâce à l'invention, il est possible ainsi de simplifier le suivi thérapeutique, d'augmenter l'efficacité et de permettre une administration plus commode pour la patiente.

L'invention constitue ainsi une garantie de sécurité dans la compliance du traitement puisqu'il devient désormais impossible que la patiente n'ingère l'estrogène sans la dose de progestatif correspondante.

Les exemples suivants sont destinés à illustrer l'invention sans pour autant présenter un caractère limitatif.

### EXEMPLE

### Préparation de capsules molles.

### Préparation de la composition.

Dans un mélange de 149 mg d'huile d'arachide de qualité Pharmacopée Européenne (FE) et de 1 mg de lécithine de soja, sont dissous 1 mg de valérate de 17β-estradiol, fournis par AKZO Diosynth, qualité Pharmacopée US, Edition XXIII (USP XXIII).

A cette solution, sont ajoutés 100 mg de progestérone micronisée - taille des particules comprise entre 1 et 100 microns -, fournis par AKZO Diosynth, de qualité PE (Pharmacopée Européenne, Ed. III). Le mélange est agité jusqu'à homogénéisation totale.

### Préparation des capsules molles.

Des capsules de gélatine contenant chacune 251 mg de la solution huileuse obtenue dans l'exemple 1.1 sont remplies selon les procédés conventionnels [Tratado de Farmacia Galénica, cité ci-dessus]. Ces capsules sont obtenues à partir d'un mélange de gélatine, glycérol, opacifiants (dioxyde de titane), colorants (quinoléine et chlorophilé cuprosodique), et eau purifiée. Tous ces ingrédients sont de qualité PE.

### EXEMPLES 2 à 5

### FORMULATIONS POUR CAPSULES "4 oval"

Les capsules de format "4 oval" ont une contenance maxi de 0,246 ml.

| | 2 | 3 | 4 | 5 |
|---|---|---|---|---|
| Progestérone | 100 mg | 100 mg | 100 mg | 100 mg |
| Estradiol valérate | 1,00 mg | 1,50 mg | 1,87 mg | 2,14 mg |
| *(Estradiol base)* | *(0,79 mg)* | *(1,18 mg)* | *(1,47 mg)* | *(1,69 mg)* |
| Huile d'arachide | 149 mg | 149 mg | 149 mg | 149 mg |
| Lécithine de soja | 1,00 mg | 1,00 mg | 1,00 mg | 1,00 mg |

### EXEMPLES 6 à 9

### FORMULATIONS POUR CAPSULES "5 oval"

Les capsules de format "5 oval" ont une contenance maxi de 0,308 ml.

| | 6 | 7 | 8 | 9 |
|---|---|---|---|---|
| Progestérone | 100 mg | 100 mg | 100 mg | 100 mg |
| Estradiol valérate | 1,41 mg | 2,11 mg | 2,63 mg | 3,01 mg |
| *(Estradiol base)* | *(1,11 mg)* | *(1,66 mg)* | *(2,07 mg)* | *(2,37 mg)* |
| Huile d'arachide | 210 mg | 210 mg | 210 mg | 210 mg |
| Lécithine de soja | 1,4 mg | 1,4 mg | 1,4 mg | 1,4 mg |

### ETUDE DE LA SOLUBILISATION DE L'ESTRADIOL DANS LES FORMULES

Les résultats ont été obtenus à partir d'une solution huile 149 mg et lécithine de soja 1 mg ("le solvant"), avec chauffage à +40°C.

| Rapport de valérate d'estradiol sur solvant (p/p) | Quantités de valérate d'estradiol dans 150 mg de solvant | Quantités d'estradiol base dans 150 mg de solvant |
|---|---|---|
| 1/70 | 2,14 mg | 1,69 mg |
| 1/80 | 1,87 mg | 1,47 mg |
| 1/100 | 1,50 mg | 1,18 mg |
| 1/150 | 1,00 mg | 0,79 mg |

### ETUDE COMPARATIVE DE LA VITESSE DE LIBERATION EN MILIEU AQUEUX DU VALERATE D'ESTRADIOL DISSOUT DANS DIFFERENTS MILIEUX LIPIDIQUES.

Le milieu aqueux utilisé est une solution aqueuse de 100ml contenant 1% de lauryl sulfate de sodium, maintenue à 37°c et agitée par rotation à 100rpm.

### Echantillons testés:

A. 5mg de valérate d'estradiol dans une composition conforme à l'invention soit 750 mg d'huile d'arachide et 500mg de progéstérone micronisé.

B. 5mg de valérate d'estradiol dans l'huile, soit 750 mg d'huile. L'estradiol est détecté en solution aqueuse par HPLC, colonne u pack C18 3,9x300mm, phases mobiles méthanol/eau, lecture par -spectrométrie UV à 280 nm.

| **Libération de valérate d'estradiol en milieu aqueux (Concentration en % du maximum théorique de 5mg/100ml, moyenne ± SD de 6 mesures)** | | |
|---|---|---|
| Temps(mn) | A | B |
| 15 | 6,2 ± 1,2 | 12,7 ± 2,6 |
| 30 | 7,3 ± 0,9 | 18,4 ± 3,4 |
| 45 | 8,0 ± 1,5 | 19,2 ± 2,8 |
| 60 | 8,9 ± 1,4 | 20,7 ± 3,2 |

Ces résultats montrent que l'addition de progéstérone en particules ralentit la libération du valérate d'estradiol dans un milieu aqueux se rapprochant de celui du tube digestif, et permet donc de contrôler la biodisponibilité des substances actives.

## Revendications

1. Composition pharmaceutique, **caractérisée par le fait qu'**elle comprend une suspension de progestérone dans un milieu lipophile contenant sous forme dissoute dans ce milieu lipophile du valérate de 17β- estradiol, le rapport en poids de progestérone au poids de 17β-estradiol base correspondant étant compris entre 25 et 600.

2. Composition la revendication 1, **caractérisée par le fait que** la progestérone est présente sous forme de particules micronisées ayant une granulométrie comprise entre 1 et 100 microns.

3. Composition selon l'une quelconque des revendications 1 ou 2, **caractérisée par le fait que** le milieu lipophile comprend une huile végétale.

4. Composition selon l'une quelconque des revendications 1 à 3, **caractérisée par le fait que** le milieu lipophile comprend en outre un agent tensio-actif.

5. Composition selon la revendication 4, **caractérisée par le fait que** l'agent tensio-actif est un agent tensio-actif amphotère.

6. Composition selon la revendication 5, **caractérisée par le fait que** l'agent tensio-actif est la lécithine de soja.

7. Composition selon l'une quelconque des revendications 4 à 6, **caractérisée par le fait que** l'agent tensio-actif est présent dans les proportions de 0,5 à 5% en poids, par rapport au poids d'huile.

8. Composition selon l'une quelconque des revendications 1 à 7, **caractérisée par le fait que** la composition contient de 50 à 300 mg de progestérone sous forme de particules et de 0,5 à 2 mg exprimé en 17β-estradiol base sous la forme de valérate de 17β-estradiol.

9. Procédé de préparation d'une composition selon l'une quelconque des revendications 1 à 8, **caractérisé par le fait que** l'on dissous dans un premier temps le valérate de 17β-estradiol dans un milieu lipophile, que l'on ajoute ensuite la progéstérone sous forme de particules et que l'on mélange la progéstérone sous forme de particules avec la solution lipophile d'estrogène.

10. Procédé selon la revendication 9 **caractérisé par le fait que** l'on chauffe le mélange de valérate d'estradiol et de milieu lipophile à une température susceptible de provoquer la dissolution totale du valérate d'estradiol.

11. Médicament **caractérisé par le fait qu'**il est formulé en vue d'une administration par voie orale ou vaginale et qu'il comprend la composition telle que définie dans l'une quelconque des revendications 1 à 8.

12. Médicament selon la revendication 11 **caractérisé par le fait qu'**il se présente sous forme de capsule molle destinée à une administration par voie orale ou vaginale.

13. Médicament selon la revendication 1 ou 12 pour son application dans le traitement hormonal substitutif de la ménopause.

14. Utilisation d'une composition selon l'une quelconque des revendications 1 à 8 pour la préparation d'un médicament destiné au traitement hormonal substitutif de la ménopause.

## Patentansprüche

1. Pharmazeutische Zusammensetzung, **dadurch gekennzeichnet, dass** sie eine Suspension von Progesteron in einem lipophilen Milieu umfasst, enthaltend in in diesem lipophilen Milieu gelöster Form 17β-Östradiolvalerat, worin das Gewichtsverhältnis von Progesteron zum Gewicht, das der 17β-Östradiol-Basis entspricht, 25 bis 600 beträgt.

2. Zusammensetzung gemäß Anspruch 1, **dadurch gekennzeichnet, dass** das Progesteron in der Form von pulverisierten Partikeln mit einer Korngröße von 1 bis 100 µm vorliegt.

3. Zusammensetzung gemäß einem der Ansprüche 1 oder 2, **dadurch gekennzeichnet, dass** das lipophile Milieu ein pflanzliches Öl umfasst.

4. Zusammensetzung gemäß einem der Ansprüche 1 bis 3, **dadurch gekennzeichnet, dass** das lipophile Milieu ausserdem ein oberflächenaktives Mittel enthält.

5. Zusammensetzung gemäß Anspruch 4, **dadurch gekennzeichnet, dass** das oberflächenaktive Mittel ein amphoteres oberflächenaktives Mittel ist.

6. Zusammensetzung gemäß Anspruch 5, **dadurch gekennzeichnet, dass** das oberflächenaktive Mittel SojaLecithin ist.

7. Zusammensetzung gemäß einem der Ansprüche 4 bis 6, **dadurch gekennzeichnet, dass** das oberflächenaktive Mittel in Mengenanteilen von 0,5 bis 5 Gew.%, bezogen auf das Gesamtgewicht des Öls, vorhanden ist.

8. Zusammensetzung gemäß einem der Ansprüche 1 bis 7, **dadurch gekennzeichnet, dass** die Zusammensetzung 50 bis 300 mg Progesteron in Form von Partikeln und 0,5 bis 2 mg, berechnet als 17β Östradiol, in Form von 17β-Östradiolvalerat enthält.

9. Verfahren zur Herstellung einer Zusammensetzung gemäß einem der Ansprüche 1 bis 8, **dadurch gekennzeichnet, dass** man zu einem ersten Zeitpunkt das 17β-Östradiolvalerat in einem lipophilen Milieu löst, man sodann das Progesteron in Form von Partikeln zugibt und das Progesteron in Form von Partikeln mit der lipophilen Östrogen-Lösung vermischt.

10. Verfahren gemäß Anspruch 9, **dadurch gekennzeichnet, dass** man die Mischung aus Östradiolvalerat und dem lipophilen Milieu auf eine Temperatur erwärmt, die geeignet ist, die vollständige Auflösung des Östradiolvalerats zu ergeben.

11. Medikament, **dadurch gekennzeichnet, dass** es für eine orale oder vaginale Verabreichung formuliert ist und die in einem der Ansprüche 1 bis 8 definierte Zusammensetzung umfasst.

12. Medikament gemäß Anspruch 11, **dadurch gekennzeichnet, dass** es in Form von Weichkapseln zur oralen oder vaginalen Verabreichung vorliegt.

13. Medikament gemäß Anspruch 11 oder 12 zur Anwendung in der substitutiven Hormon-Behandlung der Menopause.

14. Verwendung einer Zusammensetzung gemäß einem der Ansprüche 1 bis 8 zur Herstellung eines Medikaments zur substitutiven Hormon-Behandlung der Menopause.

## Claims

1. Pharmaceutical composition, **characterized in that** it comprises a suspension of progesterone in a lipophilic medium containing 17β-oestradiol valerate in dissolved form in this lipophilic medium, the ratio of the weight of progesterone to the weight of corresponding base 17β-oestradiol being between 25 and 600.

2. Composition according to Claim 1, **characterized in that** the progesterone is in the form of micronized particles having a particle size of between 1 and 100 microns.

3. Composition according to either one of Claims 1 and 2, **characterized in that** the lipophilic medium comprises a plant oil.

4. Composition according to any one of Claims 1 to 3, **characterized in that** the lipophilic medium also comprises a surfactant.

5. Composition according to Claim 4, **characterized in that** the surfactant is an amphoteric surfactant.

6. Composition according to Claim 5, **characterized in that** the surfactant is soybean lecithin.

7. Composition according to any one of Claims 4 to 6, **characterized in that** the surfactant is present in proportions of from 0.5 to 5% by weight relative to the weight of oil.

8. Composition according to any one of Claims 1 to 7, **characterized in that** the composition contains from 50 to 300 mg of progesterone in particulate form and from 0.5 to 2 mg, expressed as base 17β-oestradiol, in the form of 17β-oestradiol valerate.

9. Process for preparing a composition according to any one of Claims 1 to 8, **characterized in that**, in a first stage, the 17β-oestradiol valerate is dissolved in a lipophilic medium, **in that** the progesterone is then added in particulate form and **in that** the progesterone in particulate form is mixed with the lipophilic oestrogen solution.

10. Process according to Claim 9, **characterized in that** the mixture of oestradiol valerate and of lipophilic medium is heated to a temperature which can bring about complete dissolution of the oestradiol valerate.

11. Medicinal product, **characterized in that** it is formulated for oral or vaginal administration and **in that** it comprises the composition as defined in any one of Claims 1 to 8.

12. Medicinal product according to Claim 11, **characterized in that** it is in the form of a soft capsule intended for oral or vaginal administration.

13. Medicinal product according to Claim 11 or 12, for application in menopausal hormone replacement therapy.

14. Use of a composition according to any one of Claims 1 to 8 for the preparation of a medicinal product intended for menopausal hormone replacement therapy.
